# EUROPEAN PATENT APPLICATION

(11) **EP 4 389 737 A1**
(43) Date of publication of application: **26.06.2024**
(21) Application number: 22857631.0
(22) Date of filing: 08.08.2022
(51) Int. Cl.: C07D 233/60, H01M 10/0567, C07D 233/56

(54) **METHOD FOR PREPARING IMIDAZOLE CARBOXYLATE AND USE THEREOF**

(30) Priority: 16.08.2021 CN 202110935166
(71) Applicant: Zhangjiagang Guotai-Huarong New Chemical Materials Co., Ltd, Suzhou, Jiangsu 215600 (CN)
(72) Inventor: CHANG, Nan, Suzhou, Jiangsu 215600 (CN); SHI, Erbo, Suzhou, Jiangsu 215600 (CN); LYU, Pengcheng, Suzhou, Jiangsu 215600 (CN); YIN, Lida, Suzhou, Jiangsu 215600 (CN); ZHOU, Mingke, Suzhou, Jiangsu 215600 (CN)
(74) Representative: Bryers Intellectual Property Ltd
(86) International application number: PCT/CN2022/110823
(87) International publication number: WO 2023/020319

(57) **Abstract**

The present invention relates to a method for preparing imidazole carboxylate and the use thereof, and mainly solves the technical problems of the complex preparation process and cumbersome operation of imidazole carboxylate, and the low yield and low purity of the prepared imidazole carboxylate. According to the present invention, imidazole carboxylate is generated by reacting imidazole formyl chloride and alcohol in the presence of a first solvent and an organic base, and the imidazole carboxylate having a high yield and a high purity is further obtained by means of a post-treatment, which has the advantages of a simple preparation method, mild preparation conditions and strong operability.

## Description

### Technical Field of the Invention

The present disclosure relates to a method for preparing imidazole carboxylate and use thereof.

### Background of the Invention

In recent years, with the increasing awareness of environmental protection among people, research on high-energy chemical power sources around the world has been continuously deepening around the issue of green and sustainable energy development, where lithium-ion batteries have become one of the most practical and advantageous types of power sources.

Electrolyte solution is an important component of lithium-ion batteries. In order to improve the performance of lithium-ion batteries, different additives are usually added to the electrolyte solution. Due to the complex internal reactions during the operation of lithium-ion batteries, it is necessary to add additives with high purity to avoid impurities affecting the operation of lithium-ion batteries.

Imidazole carboxylate can be used as an additive in electrolyte solutions to improve the performance of lithium-ion batteries, but there is currently no preparation method for imidazole carboxylate with simple process steps, mild reaction conditions, high yield, and high purity.

### Summary of the Invention

The purpose of the present disclosure is to provide a method for preparing imidazole carboxylate with simple process steps, mild reaction conditions, high yield, and high purity.

Another purpose of the present disclosure is to provide use of imidazole carboxylate prepared by the above preparation method in the electrolyte solution of lithium-ion batteries.

To achieve the above purpose, a technical solution employed by the present disclosure is as follows:
In one aspect, the present disclosure provides a method for preparing imidazole carboxylate, which comprises reacting imidazole formyl chloride with an alcohol in the presence of a first solvent and an organic base to give imidazole carboxylate.

Preferably, the alcohol has a chemical structure formula represented by formula (1), formula (1) is R-OH, R in formula (1) is hydrocarbyl.

Preferably, the imidazole carboxylate has a chemical structure formula represented by formula (2), formula (2) is 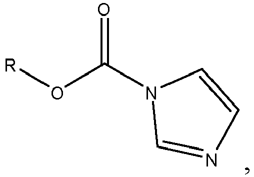 R in formula (2) is hydrocarbyl.

Wherein, the reaction equation for the reaction between imidazole formyl chloride and the alcohol is

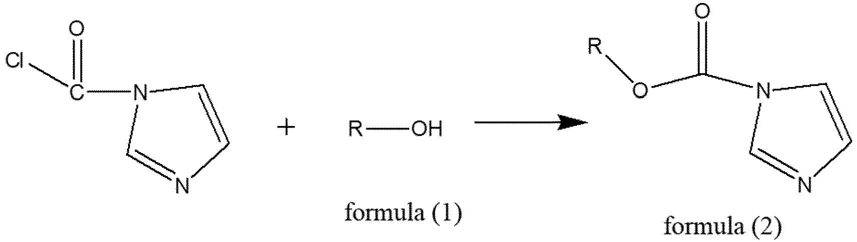

Further preferably, R is hydrocarbyl with 1 - 5 carbon atoms, more further a linear hydrocarbyl with 1 - 5 carbon atoms.

Further preferably, R is one selected from the group consisting of propynyl, butynyl, pentynyl, propenyl, butenyl, pentenyl, methyl, ethyl, propyl, butyl, and pentyl.

Preferably, the molar ratio of the alcohol to imidazole formyl chloride is (0.8 - 0.9): 1.

Preferably, the alcohol and the organic base are added dropwise in the form of a mixture. The use of dropwise addition can effectively control the reaction rate between the alcohol and imidazole formyl chloride, ensuring as much as possible the complete reaction between the alcohol and imidazole formyl chloride.

Further preferably, the temperature of dropwise addition is controlled to be 0 ∼ 30 °C.

Further preferably, the rate of dropwise addition is controlled at 2 ∼ 8 g/min, more further 4 - 6 g/min.

Preferably, the first solvent is selected from the group consisting of toluene, ethyl acetate, dichloromethane, dichloroethane, and combinations thereof.

Further preferably, when the alcohol is a saturated alcohol, the first solvent is selected from the group consisting of dichloromethane, dichloroethane, toluene, and combinations thereof, when the alcohol is an unsaturated alcohol, the first solvent is selected from the group consisting of dichloromethane, ethyl acetate, dichloroethane, toluene, and combinations thereof.

Further preferably, the mass ratio of the first solvent to imidazole formyl chloride is (20 ∼ 25): 1.

Preferably, the organic base is selected from the group consisting of triethylamine, pyridine, imidazole, and combinations thereof.

Further preferably, the molar ratio of the organic base to imidazole formyl chloride is (0.95 - 1.05): 1.

Preferably, the reaction temperature is controlled to be 0 ∼ 30 °C, further 0 ~ 20 °C, more further 0 ∼10 °C.

Preferably, the reaction time is controlled to be 1 ∼ 3 h, more further 1 - 2 h.

Preferably, the preparation method further comprises a step of post-processing the reaction solution after the reaction, and the post-processing comprises steps of first mixing the reaction solution with water, standing, layering, and collecting the organic phase, then dehydrating the organic phase to remove water from the organic phase, then removing some of the first solvent in the organic phase and then mixing with a second solvent, crystallizing, filtering, and drying. By using the way of mixing the second solvent and the organic phase to crystallize, the imidazole carboxylate dissolved in the organic phase is precipitated, and the purity of the imidazole carboxylate product prepared by this method is high.

Further preferably, the removed first solvent accounts for 83% ~ 93% of the total mass of the first solvent.

Further preferably, the second solvent is selected from the group consisting of petroleum ether, n-hexane, cyclohexane, and combinations thereof.

More further preferably, the mass ratio of the second solvent to imidazole formyl chloride is (4.5 - 5.5): 1, more further (4.5 - 5.0): 1.

Further preferably, the temperature of mixing and crystallization is controlled to be -20 ~ 0 °C.

According to some preferred implementations, the preparation method comprises steps of:
(1) mixing the first solvent with imidazole formyl chloride under nitrogen protection to prepare a mixture, and adding a mixture of the alcohol and the organic base dropwise to the mixture to allow imidazole formyl chloride to react with the alcohol at 0 - 30 °C for 1 ∼ 3 h, wherein the dropwise rate is controlled at 2 ∼ 8 g/min;
(2) mixing the reaction solution after reaction in step (1) with water for washing, allowing the reaction solution containing water after washing to stand and layer, and collecting the organic phase, wherein the feeding mass of water is 15 ~ 30% of the total feeding mass of the first solvent, imidazole formyl chloride, the alcohol, and the organic base;
(3) mixing the organic phase with a dehydrating agent to dehydrate the organic phase, wherein the dehydrating agent is selected from the group consisting of anhydrous sodium sulfate, anhydrous magnesium sulfate, anhydrous calcium chloride, and combinations thereof;
(4) evaporating the dehydrated organic phase under vacuum to remove some of the first solvent, then mixing with the second solvent and crystallizing, filtering, and drying to give an imidazole carboxylate finished product, wherein the removed first solvent accounts for 83% ~ 93% of the total mass of the first solvent, and the mass ratio of the second solvent to imidazole formyl chloride is (4.0 - 5.5): 1, and the temperature of mixing and crystallization is controlled to -20 ~ 0 °C.

The present disclosure optimizes the preparation process of imidazole carboxylate, enabling the prepared imidazole carboxylate to have both high yield and high purity, with a yield of over 92% and a purity of over 99.5%, which can be directly applied as an additive in the electrolyte solution of lithium-ion batteries.

Further preferably, in step (2), the feeding mass of water is 20 ~ 30% of the total feeding mass of the first solvent, imidazole formyl chloride, the alcohol, and the organic base.

Another aspect of the present disclosure provides use of imidazole carboxylate prepared by the above preparation method in the electrolyte solution of lithium-ion batteries.

Preferably, the addition amount of imidazole carboxylate is 0.1 ∼ 2.0%, further 0.1 ∼ 0.5% of the total mass of the electrolyte solution.

Due to the use of the above technical solutions, the present disclosure has the following advantages over the prior art:
The preparation method of imidazole carboxylate in the present disclosure has a simple process route, mild reaction conditions, and strong operability, and the prepared imidazole carboxylate can have both high yield and high purity, and can be used as an additive in the electrolyte solution of lithium-ion batteries.

### Brief Description of the Drawings

Figure 1 shows a nuclear magnetic resonance hydrogen spectrum of the product in Embodiment 1 of the present disclosure;
Figure 2 shows a nuclear magnetic resonance carbon spectrum of the product in Embodiment 1 of the present disclosure.

### Detailed Description of Exemplary Embodiments

The present disclosure is further described below combining with embodiments. However, the present disclosure is not limited to the following embodiments. The implementation conditions used in the embodiments may be further adjusted according to different requirements of specific use, and undefined implementation conditions usually are conventional conditions in the industry. The technical features involved in the various implementations described below may be combined with each other if they do not conflict with each other.

### Embodiment 1

Under the protection of nitrogen, 2600 g dichloromethane and 130 g imidazole formyl chloride were mixed and stirred, and a mixture of 50 g propargyl alcohol and 101 g triethylamine was added dropwise at 0 - 10 °C, with a dropping rate of 5 g/min, stirring while adding dropwise, and after dropping, the reaction was carried out at 0 - 10 °C for 2 h.

After the reaction was completed, 576 g of deionized water was added to the reaction solution to wash the reaction solution, and after washing, the organic phase was extracted by standing stratification, and 200 g of anhydrous sodium sulfate was added to the organic phase to dehydrate the organic phase.

After dehydration, 2200 g of dichloromethane was removed by vacuum evaporation from the organic phase, then 600 g of petroleum ether was added to the remaining organic phase for crystallization at -20 °C, and after crystallization, the solution was filtered to collect a solid, which was dried to give the finished product.

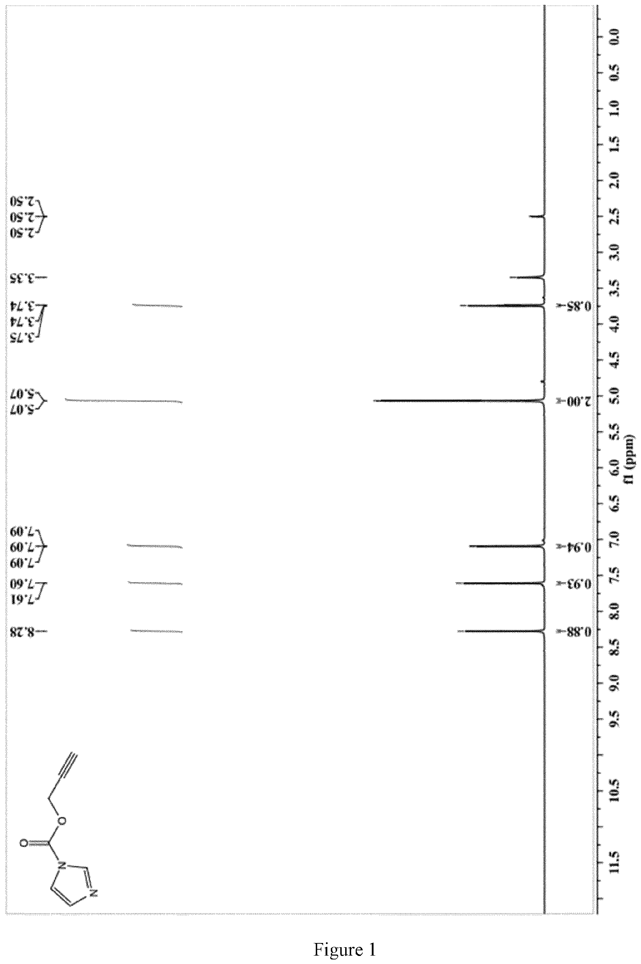

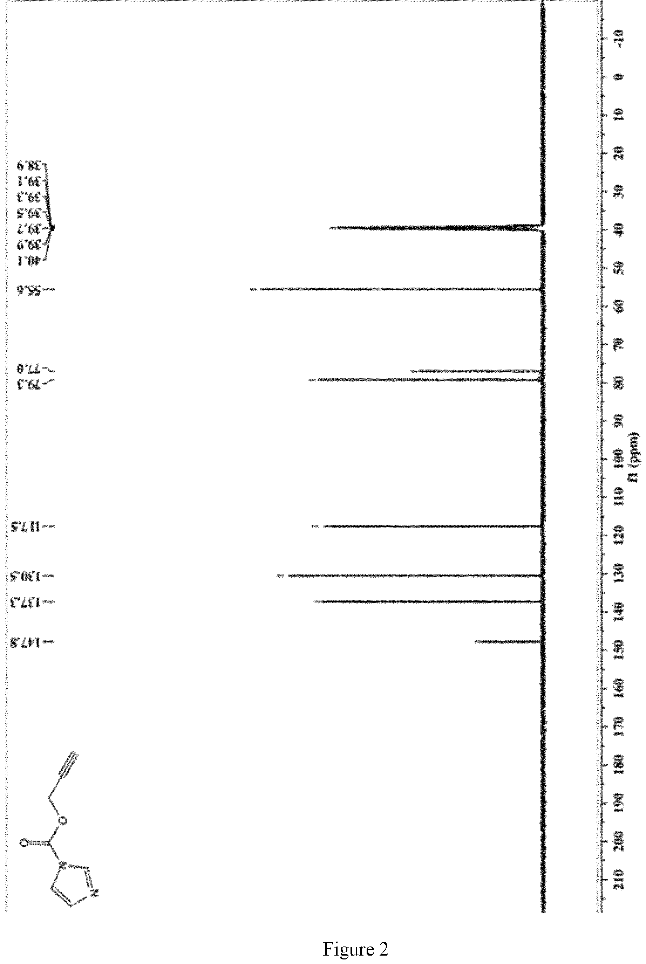

The finished product had a weighed mass of 127 g and a yield of 95%. The product was characterized using nuclear magnetic resonance, and the relevant characterization spectra are shown in Figures 1 and 2. The results confirmed that it was the target product, propynyl imidazole carboxylate, with a purity of 99.8%.

### Embodiments 2 to 15

They adopted the preparation method of Embodiment 1 to prepare imidazole carboxylates, and the differences between them and Embodiment 1 are shown in Table 1.

**Table 1**

| Embodiments | First solvents | Mass ratio of imidazole formyl chloride to the first solvent | Structure of alcohols | Organic bases | Molar ratio of imidazole formyl chloride to alcohol and organic base | Reaction temperature (°C) | Reaction time (h) | Ratio of feeding deionized water to the total mass of reaction solution | Second solvent | Products | Purity (%) | Yield (%) |
|---|---|---|---|---|---|---|---|---|---|---|---|---|
| Embodiment 1 | Dichloromethane | 1: 20 | 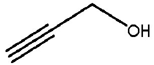 | Triethylamine | 1: 0.9: 1 | 0 ∼ 10 | 2 | 0.2 | Petroleum ether | 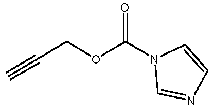 | 99.8 | 95 |
| Embodiment 2 | Ethyl acetate | 1: 22 | 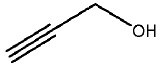 | Triethylamine | 1: 0.9: 1 | 0 ∼ 10 | 2 | 0.2 | n-hexane | 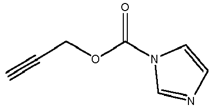 | 99.9 | 96 |
| Embodiment 3 | Dichloroethane | 1: 20 | 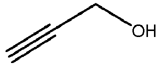 | Imidazole | 1: 0.9: 1 | 0 ∼ 10 | 2 | 0.3 | n-hexane | 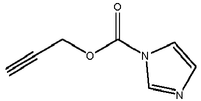 | 99.8 | 94 |
| Embodiment 4 | Toluene | 1: 25 | 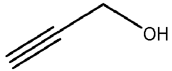 | Pyridine | 1: 0.8: 1 | 10 ~ 20 | 3 | 0.25 | Petroleum ether | 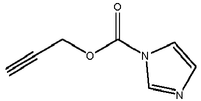 | 99.8 | 95 |
| Embodiment 5 | Dichloromethane | 1:23 | 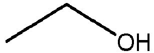 | Imidazole | 1: 0.8: 1 | 10 ~ 20 | 1 | 0.25 | n-hexane | 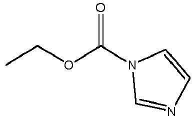 | 99.6 | 97 |
| Embodiment 6 | Dichloromethane | 1: 25 | 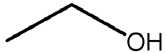 | Pyridine | 1: 0.8: 1 | 10-20 | 1 | 0.25 | Cyclohexane | 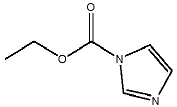 | 99.6 | 93 |
| Embodiment 7 | Dichloroethane | 1: 21 | 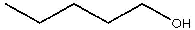 | Triethylamine | 1: 0.9: 1 | 20 ∼ 30 | 1 | 0.3 | Cyclohexane | 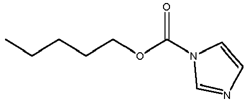 | 99.7 | 92 |
| Embodiment 8 | Dichloroethane | 1: 24 | 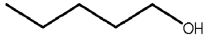 | Triethylamine | 1: 0.9: 1 | 20 ∼ 30 | 1 | 0.3 | Petroleum ether | 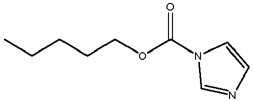 | 99.7 | 93 |
| Embodiment 9 | Toluene | 1: 25 | 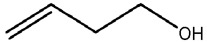 | Pyridine | 1: 0.85: 1 | 20 ∼ 30 | 3 | 0.2 | Petroleum ether | 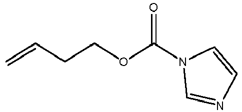 | 99.5 | 95 |
| Embodiment 10 | Ethyl acetate | 1: 24 | 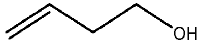 | Pyridine | 1: 0.8: 1 | 10 ~ 20 | 2 | 0.25 | n-hexane | 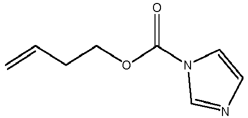 | 99.6 | 95 |
| Embodiment 11 | Toluene | 1: 25 | 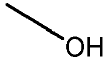 | Imidazole | 1: 0.9: 1 | 0 ∼ 10 | 1 | 0.2 | Petroleum ether | 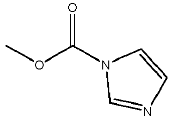 | 99.8 | 94 |
| Embodiment 12 | Toluene | 1: 20 | 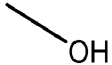 | Imidazole | 1: 0.9: 1 | 0 ∼ 10 | 1 | 0.2 | Cyclohexane | 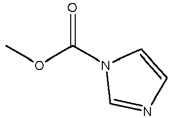 | 99.7 | 97 |
| Embodiment 13 | Dichloromethane | 1:20 | 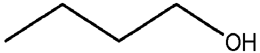 | Imidazole | 1: 0.8: 1 | 10 ∼ 20 | 1 | 0.2 | Petroleum ether | 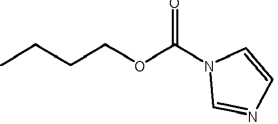 | 99.8 | 96 |
| Embodiment 14 | Toluene | 1: 20 | 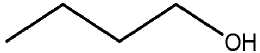 | Triethylamine | 1: 0.9: 1 | 0 ∼ 10 | 1 | 0.2 | n-hexane | 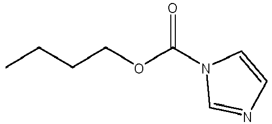 | 99.5 | 93 |
| Embodiment 15 | Toluene | 1: 20 | 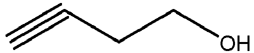 | Triethylamine | 1: 0.9: 1 | 0 ∼ 10 | 1 | 0.2 | n-hexane | 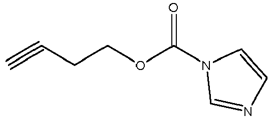 | 99.6 | 94 |

### Embodiment 16

Under the protection of nitrogen, 2600 g dichloromethane and 130 g imidazole formyl chloride were mixed and stirred, and a mixture of 50 g propargyl alcohol and 101 g triethylamine was added dropwise at 0 - 10 °C, with a dropping rate of 5 g/min, stirring while adding dropwise, and after dropping, the reaction was carried out at 0 - 10 °C for 2 h.

After the reaction was completed, 618 g of deionized water was added to the reaction solution to wash the reaction solution, and after washing, the organic phase was extracted by standing stratification, and 200 g of anhydrous sodium sulfate was added to the organic phase to dehydrate the organic phase.

After dehydration, 2100 g of dichloromethane was removed by vacuum evaporation from the organic phase, then 600 g of petroleum ether was added to the remaining organic phase for crystallization at -20 °C, and after crystallization, the solution was filtered to collect a solid, which was dried to give the finished product.

The finished product had a weighed mass of 70 g and a yield of 53%. The product was characterized using nuclear magnetic resonance, and the results confirmed that it was the target product, propynyl imidazole carboxylate, with a purity of 99.5%.

Compared with Embodiment 1, the product yield of this embodiment is lower.

### Embodiment 17

Under the protection of nitrogen, 2600 g dichloromethane and 130 g imidazole formyl chloride were mixed and stirred, and a mixture of 50 g propargyl alcohol and 101 g triethylamine was added dropwise at 0 - 10 °C, with a dropping rate of 5 g/min, stirring while adding dropwise, and after dropping, the reaction was carried out at 0 - 10 °C for 2 h.

After the reaction was completed, 618 g of deionized water was added to the reaction solution to wash the reaction solution, and after washing, the organic phase was extracted by standing stratification, and 200 g of anhydrous sodium sulfate was added to the organic phase to dehydrate the organic phase.

After dehydration, 2500 g of dichloromethane was removed by vacuum evaporation from the organic phase, then 600 g of petroleum ether was added to the remaining organic phase for crystallization at -20 °C, and after crystallization, the solution was filtered to collect a solid, which was dried to give the finished product.

The finished product had a weighed mass of 130 g and a yield of 97%. The product was characterized using nuclear magnetic resonance, and the results confirmed that it was the target product, propynyl imidazole carboxylate, with a purity of 98.5%.

Compared with Embodiment 1, the product purity of this embodiment is lower.

### Comparative example 1

276 g of CDI (N,N'- carbonyl diimidazole) was added to 3795 ml of dichloromethane, a mixture of propargyl alcohol and dichloromethane was added dropwise in an ice water bath (the mixture was prepared by dissolving 56 g of propargyl alcohol in 678 ml of dichloromethane), the solution was stirred for 1 h, heated to room temperature, and stirred for 12 h, and after the reaction was completed, the solution was washed three times with 1200 ml of water to collect the organic phase, the organic phase was dried with anhydrous magnesium sulfate, filtered and concentrated under vacuum to give a colorless liquid of 138 g.

Wherein, the main component of the colorless liquid was 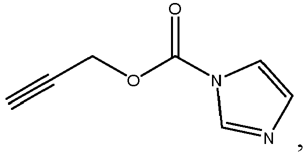 with a yield of 92% and a purity of 97.9%.

Compared with Embodiment 1, the purity of the product of Comparative example 1 is insufficient to meet the demand for electrolyte additives, and more wastewater is generated that does not meet environmental requirements.

### Comparative example 2

56 g of propargyl alcohol was used as the raw material, cooled to -8 °C, phosgene was introduced into the reactor at a rate of 50 ml/min, the reaction temperature was controlled to lower than 5 °C, after the reaction was completed, the temperature of the solution was maintained for 30 min, and then nitrogen gas was introduced at room temperature for 1.5 h, the fraction at 118 °C under vacuum distillation was collected, 174 ml of dichloromethane and 75 g of imidazole were added to the reactor, and 106 ml of triethylamine was added at a rate of 100 ml/min, and after the addition, the gas in the reactor was replaced with nitrogen gas, the temperature was heated to reflux temperature, then the above fraction was added dropwise to the reactor for 1 h, the system reacted for 5 h, and was suction filtered, the filter cake was washed with dichloromethane, the separated mother liquor was washed with an alkaline solution to pH 7.3, dried with anhydrous magnesium sulfate, and distilled under vacuum to give 141 g of product, with a yield of 94% and a purity of 99.4%.

Compared with Embodiment 1, Comparative example 2 requires the use of phosgene as the raw material, which is highly toxic and difficult to operate.

### Application example 1

The imidazole carboxylate prepared in Embodiment 1 was applied to the electrolyte solution, and the specific implementation was as follows:
In a glove box filled with argon gas (H₂O content < 10 ppm), EC, DMC, and EMC were mixed evenly in a volume ratio of 1: 1: 1, 1 mol/L of LiPF₆ was added to the mixed solution, and then 0.3 wt% of prop-2-yn-1-yl-1H-imidazol-1-carboxylate was added to the solution to prepare an electrolyte solution.

### Application example 2

In a glove box filled with argon gas (H₂O content < 10 ppm), EC, DMC, and EMC were mixed evenly in a volume ratio of 1: 1: 1, and 1mol/L of LiPF₆ was added to the mixed solution to prepare an electrolyte solution.

The electrolyte solutions prepared in Application Example 1 and Application Example 2 were respectively tested for capacity retention ratio after 4 H of storage at a high temperature of 85 °C and capacity retention ratio after 300 cycles at 45 °C in a 4.2V lithium cobalt oxide graphite battery, and the relevant experimental data are shown in Table 2.

**Table 2**

| | Capacity retention ratio after 4 H of storage at a high temperature of 85 °C (%) | Capacity retention ratio after 300 cycles at 45 °C (%) | Battery bulge ratio after 300 cycles at 45 °C (%) |
|---|---|---|---|
| Application example 1 | 91.4 | 93.0 | 3.5 |
| Application example 2 | 85.3 | 85.3 | 4.2 |

The above detailed describes the present disclosure, and is intended to make those skilled in the art being able to understand the present disclosure and thereby implement it, and should not be concluded to limit the protective scope of this disclosure. Any equivalent variations or modifications according to the spirit of the present disclosure should be covered by the protective scope of the present disclosure.

## Claims

1. A method for preparing imidazole carboxylate, **characterized in that,** reacting imidazole formyl chloride with an alcohol in the presence of a first solvent and an organic base to give imidazole carboxylate.

2. The method for preparing imidazole carboxylate according to claim 1, **characterized in that,** the alcohol has a chemical structure formula represented by formula (1), formula (1) is R-OH, R in formula (1) is hydrocarbyl; and/or, the molar ratio of the alcohol to imidazole formyl chloride is (0.8 - 0.9): 1.

3. The method for preparing imidazole carboxylate according to claim 1, **characterized in that,** the imidazole carboxylate has a chemical structure formula represented by formula (2), formula (2) is 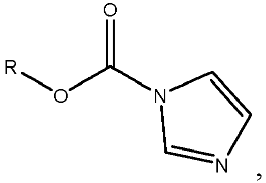 R in formula (2) is hydrocarbyl.

4. The method for preparing imidazole carboxylate according to claim 2, **characterized in that,** R is hydrocarbyl with 1 - 5 carbon atoms.

5. The method for preparing imidazole carboxylate according to claim 4, **characterized in that,** R is one selected from the group consisting of propynyl, butynyl, pentynyl, propenyl, butenyl, pentenyl, methyl, ethyl, propyl, butyl, and pentyl.

6. The method for preparing imidazole carboxylate according to claim 1, **characterized in that,** the alcohol and the organic base are added dropwise in the form of a mixture, the temperature of dropwise addition is controlled to be 0 ∼ 30 °C, and/or, the rate of dropwise addition is controlled at 2 ∼ 8 g/min.

7. The method for preparing imidazole carboxylate according to claim 1, **characterized in that,** the first solvent is selected from the group consisting of toluene, ethyl acetate, dichloromethane, dichloroethane, and combinations thereof, and the mass ratio of the first solvent to imidazole formyl chloride is (20 ∼ 25): 1.

8. The method for preparing imidazole carboxylate according to claim 1, **characterized in that,** the organic base is selected from the group consisting of triethylamine, pyridine, imidazole, and combinations thereof, and the molar ratio of the organic base to imidazole formyl chloride is (0.95 - 1.05): 1.

9. The method for preparing imidazole carboxylate according to claim 1, **characterized in that,** the reaction temperature is controlled to be 0 ∼ 30 °C, and/or, the reaction time is controlled to be 1 ∼ 3 h.

10. The method for preparing imidazole carboxylate according to claim 1, **characterized in that,** the preparation method further comprises a step of post-processing the reaction solution after the reaction, and the post-processing comprises steps of first mixing the reaction solution with water, standing, layering, and collecting the organic phase, then dehydrating the organic phase to remove water from the organic phase, then removing some of the first solvent in the organic phase and then mixing with a second solvent, crystallizing, filtering, and drying.

11. The method for preparing imidazole carboxylate according to claim 10, **characterized in that,** the removed first solvent accounts for 83% ∼ 93% of the total mass of the first solvent.

12. The method for preparing imidazole carboxylate according to claim 10, **characterized in that,** the second solvent is selected from the group consisting of petroleum ether, n-hexane, cyclohexane, and combinations thereof, and the mass ratio of the second solvent to imidazole formyl chloride is (4.5 ∼ 5.5): 1.

13. The method for preparing imidazole carboxylate according to claim 1, **characterized in that,** the preparation method comprises steps of:
(1) mixing the first solvent with imidazole formyl chloride under nitrogen protection to prepare a mixture, and adding a mixture of the alcohol and the organic base dropwise to the mixture to allow imidazole formyl chloride to react with the alcohol at 0 ∼ 30 °C for 1 ∼ 3 h, wherein the dropwise rate is controlled at 2 ∼ 8 g/min;
(2) mixing the reaction solution after reaction in step (1) with water for washing, allowing the reaction solution containing water after washing to stand and layer, and collecting the organic phase, wherein the feeding mass of water is 15 ~ 30% of the total feeding mass of the first solvent, imidazole formyl chloride, the alcohol, and the organic base;
(3) mixing the organic phase with a dehydrating agent to dehydrate the organic phase, wherein the dehydrating agent is selected from the group consisting of anhydrous sodium sulfate, anhydrous magnesium sulfate, anhydrous calcium chloride, and combinations thereof;
(4) evaporating the dehydrated organic phase under vacuum to remove some of the first solvent, then mixing with the second solvent and crystallizing, filtering, and drying to give an imidazole carboxylate finished product, wherein the removed first solvent accounts for 83% ~ 93% of the total mass of the first solvent, and the mass ratio of the second solvent to imidazole formyl chloride is (4.0 ∼ 5.5): 1, and the temperature of mixing and crystallization is controlled to -20 ∼ 0 °C.

14. Use of imidazole carboxylate prepared by the preparation method according to claim 1 in the electrolyte solution of lithium-ion batteries.

15. The use of imidazole carboxylate in the electrolyte of lithium-ion batteries according to claim 14, **characterized in that**, the addition amount of imidazole carboxylate is 0.1 ~ 2.0% of the total mass of the electrolyte solution.
